# EUROPÄISCHE PATENTSCHRIFT

(11) **EP 1 718 240 B1**
(45) Veröffentlichungstag und Bekanntmachung des Hinweises auf die Patenterteilung: **04.07.2007**
(21) Anmeldenummer: 05707636.6
(22) Anmeldetag: 25.02.2005
(51) Int. Cl.: A61F 2/00

(54) **HERNIENNETZ ZUR VERSORGUNG VON LEISTEN- ODER HIATUSHERNIEN**
HERNIA NET FOR TREATING INGUINAL OR HIATUS HERNIAS
FILET HERNIAIRE SERVANT A SOIGNER DES HERNIES INGUINALES OU HIATALES

(30) Priorität: 26.02.2004 DE 102004009894
(43) Veröffentlichungstag der Anmeldung: 08.11.2006
(73) Patentinhaber: GfE Medizintechnik GmbH, 90431 Nürnberg (DE)
(72) Erfinder: KÖCKERLING, Ferdinand, 30177 Hannover (DE); ZIMMERMANN, Hanngörg, 91327 Gössweinstein (DE); HEINLEIN, Markus, 91327 Gössweinstein (DE)
(74) Vertreter: Hübner, Gerd
(86) Internationale Anmeldenummer: PCT/EP2005/002029
(87) Internationale Veröffentlichungsnummer: WO 2005/082274

(56) Entgegenhaltungen:
- WO-A-96/03091
- DE-A1- 19 832 634
- FR-A- 2 744 906
- US-A1- 2002 001 609
- US-A1- 2002 013 590
- US-A1- 2003 171 823
- US-A1- 2003 212 460

## Beschreibung

Die Erfindung betrifft ein Herniennetz zur Versorgung von insbesondere Leisten- oder Hiatushernien mit den im Oberbegriff des Anspruches 1 angegebenen Merkmalen.

Derartige Herniennetze sind hinsichtlich ihrer grundsätzlichen Konfiguration in der Medizintechnik üblich und Standardprodukte für die Hemienversorgung. Eine spezielle Weiterbildung auf der Basis dieser Grundkonfiguration ist beispielsweise der WO 00/67663 A1 zu entnehmen.

Herniennetze werden bei der operativen Reparation von insbesondere Leistenhemien verwendet, um ein spannungsfreies Überdecken des Defektes zur Stabilisierung der Bauchwand zu erzielen. Je nach Art und Lage der Hernie kann es dabei notwendig sein, eine Körperröhre, wie beispielsweise den Samenstrang bei einer Leistenhernie oder die Speiseröhre bei einer Hiatushernie durch das Netz zu führen. Hierzu ist in der so bezeichneten Grundplatte aus einem lagenförmigen, flexiblen Netzmaterial eine Durchlassöffnung angelegt. Da die Körperröhre naturgemäß keinen in die Durchlassöffnung einfädelbaren Anfang besitzt, muss in der Grundplatte ein Einführungsschlitz zwischen dem äußeren Umriss der Grundplatte und der Durchlassöffnung zum Einführen der Körperröhre dorthin angelegt sein.

In der herkömmlichen Operationstechnik wird dieser Schlitz nach dem Einführen der Körperröhre in die Durchlassöffnung geschlossen, indem die Schlitzflanken in eine Überlapp-Stellung gebracht und miteinander vernäht werden. Dieses Zusammenziehen sorgt allerdings für eine Deformation des Herniennetzes, was eine saubere Lage an der Bauchwand oder am Zwerchfell beeinträchtigen kann.

Zur Lösung der vorstehenden Problematik ist es laut der FR-A-2744906 nun vorgesehen, das Herniennetz im Bereich der Mündung des Einführungsschlitzes mit einer Nähbrücke zu versehen, die über den Einführungsschlitz klappbar und beiderseits davon mit dem Netzmaterial der Grundplatte vernähbar ist. Dank dieser Nähbrücke können die den Einführungsschlitz flankierenden Zuschnittslappen des Herniennetzes in einer Ebene und glatt liegen bleiben und trotzdem miteinander vernäht werden. Ein Aufbauschen und Deformieren des Herniennetzes werden vermieden, sodass es sauber an Bauchwand bzw. Zwerchfell zu liegen kommen kann.

Problematisch bei diesem Stand der Technik ist die aufwendige Handhabung der separaten Nähbrücke, was einen zusätzlichen Herstellungsaufwand mit sich bringt. Die Erfindung schlägt daher laut kennzeichnungsteil des Anspruches 1 die einstückige Ausbildung der Nähbrücke aus dem Netzmaterial der Grundplatte von Dadurch erübrigt sich eine umständliche Handhabung kleinflächiger Netzteile zum Zuschneiden und Fixieren an dem eigentlichen Herniennetz.

Weitere bevorzugte Ausbildungen eines solchen Herniennetzes sind in den Unteransprüchen angegeben. Merkmale, Einzelheiten und Vorteile der Erfindung ergeben sich ferner aus der nachfolgenden Beschreibung, in der Ausführungsbeispiele anhand der beigefügten Zeichnungen näher erläutert werden. Es zeigen:
- Fig. 1: eine Draufsicht auf ein Herniennetz für eine Hiatushernie,
- Fig. 2: eine Draufsicht auf ein Herniennetz für eine Leistenhernie,
- Fig. 3 und 4: Draufsichten auf Herniennetze für eine Hiatus- beziehungsweise Leistenhernie in einer zu den Fig. 1 beziehungsweise 2 alternativen Ausführungsform, und
- Fig. 5A-D: eine schematische Abfolge von Herstellungsschritten für das Herniennetz gemäß Fig. 3.

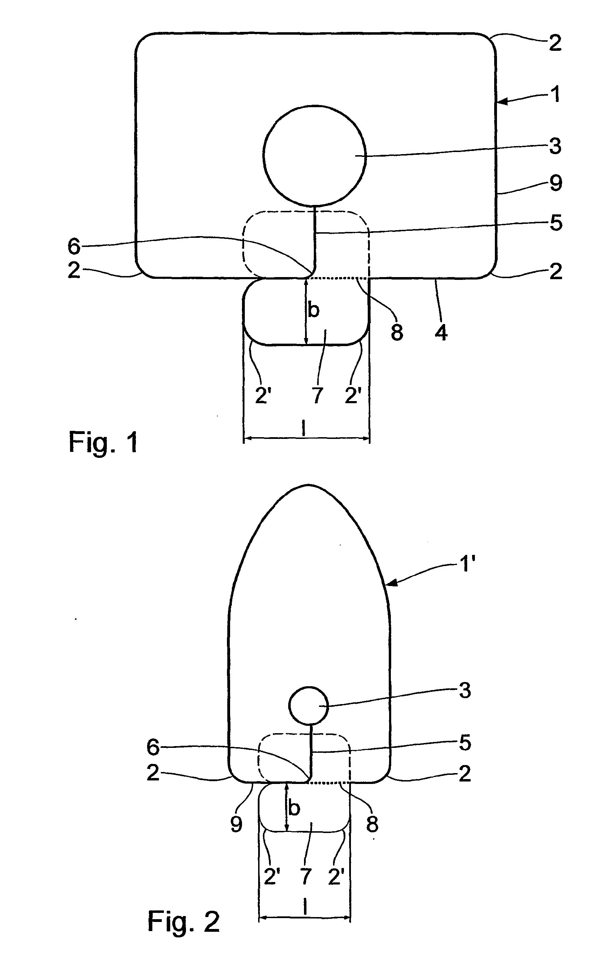

Das in Fig. 1 gezeigte Herniennetz dient zur Versorgung einer Hiatushernie. Es weist eine Grundplatte 1 aus einem üblichen lagenförmigen, flexiblen Netzmaterial auf, das aus einem monofilen Polypropylen-Faden in Atlas-Legung gewirkt ist. Das Flächengewicht des Herniennetzes kann zwischen 60 und 65 g/m² betragen, aber auch deutlich darunter liegen.

Die äußere Umrissform der Grundplatte 1 ist im Wesentlichen rechteckig mit abgerundeten Eckbereichen 2. Zentral ist eine Durchlassöffnung 3 angelegt, die im Falle des gezeigten Hiatus-Herniennetzes zur Durchführung der Speiseröhre dient. Ausgehend von dieser Durchlassöffnung 3 verläuft zur einen Längsseite 4 der Grundplatte 1 hin ein gerader Einführungsschlitz 5, der mit einer gebogenen Mündung 6 in diese Längsseite 4 an zentraler Position übergeht. Auf einer Seite neben der Mündung 6 ist an der Längsseite 4 der Grundplatte 1 eine einstückig angesetzte, im Wesentlichen etwa rechteckförmige Brückenlasche 7 an den Zuschnitt der Grundplatte 1 angehängt. Auch die Eckbereiche 2' der Brückenlasche 7 sind abgerundet. Die Breite b der Brückenlasche 7 entspricht knapp der Länge des Einführungsschlitzes 5. Ihre Länge 1 entspricht etwa der doppelten Breite b.

Die in Fig. 1 mit durchgezogenen Linien dargestellte Form der Grundplatte 1, der Durchlassöffnung 3, des Einführungsschlitzes 5 und der Nähbrücke 7 wird mit Hilfe eines Laser-Schneidstrahls aus einem Netz-Bahnmaterial hergestellt. Durch das Laserschneiden sind die Schneidkanten sauber abgeschmolzen, sodass keine Faserstücke aus dem Herniennetz auszutreten drohen.

Beim Einsatz des Herniennetzes wird es durch Öffnen des Einführungsschlitzes 5 über die Speiseröhre gezogen, bis diese in der Durchlassöffnung 3 zu liegen kommt. Die Grundplatte 1 wird mit den Flanken des Einführungsschlitzes 5 sauber aneinanderliegend geglättet und anschließend die Brückenlasche 7 entlang der punktierten, in der Flucht der verbleibenden Längsseite 4 liegenden Faltkante 8 nach innen umgeschlagen, sodass der Einführungsschlitz 5 bis kurz vor der Durchlassöffnung 3 und in Längsrichtung beiderseits im Wesentlichen symmetrisch überdeckt wird. Anschließend werden - wie nicht näher dargestellt ist - die Brückenlasche 7 mit den darunter befindlichen Teilen der Grundplatte 1 beiderseits des Einführungsschlitzes 5 vernäht, sodass der Schlitz 5 stabil geschlossen ist.

Das in Fig. 2 dargestellte Herniennetz dient zur Versorgung einer Leistenhernie. Seine Grundplatte 1' ist im Wesentlichen torbogenförmig, wobei im unteren, rechteckigen Teil die Durchlassöffnung 3 und der Einführungsschlitz 5 im Zuschnitt angelegt sind. Letzterer mündet in die kurze Querseite 9 der Grundplatte 1'. Analog der Ausführungsform gemäß Fig. 1 ist wiederum anschließend an die Mündung 6 des Schlitzes 5 eine Brückenlasche 7 einstückig angesetzt. Nach dem Positionieren des Herniennetzes so, dass der Samenstrang durch die Durchlassöffnung 3 verläuft, kann der Einführungsschlitz 5 wie anhand von Fig. 1 beschrieben durch Umlegen der Brückenlasche 7 entlang der Faltkante 8 und Vernähen mit den beiderseitigen Bereichen entlang der Schlitzflanken geschlossen werden.

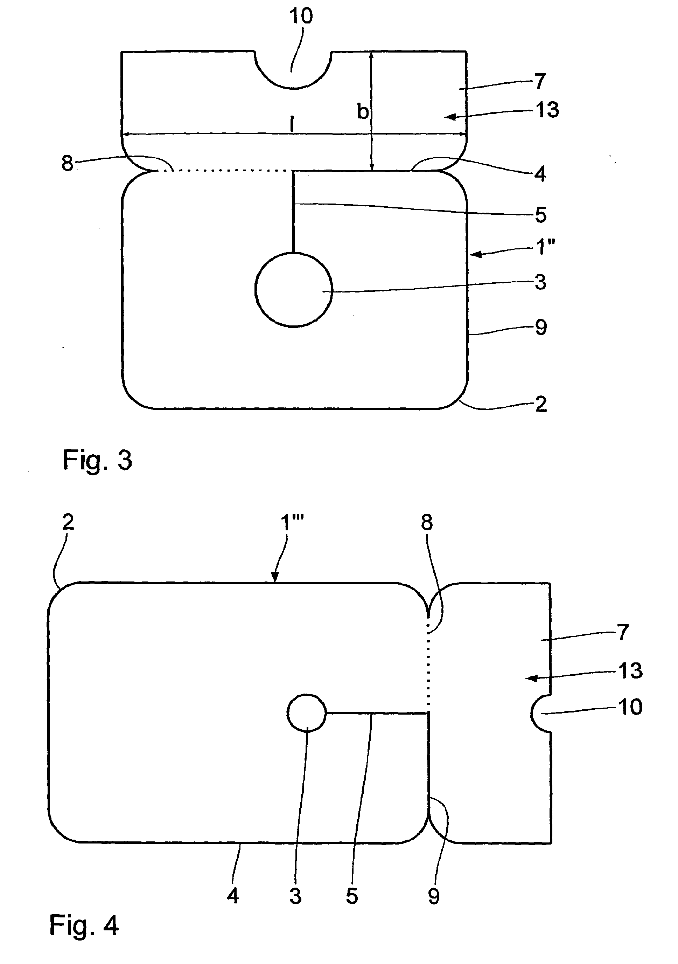

Das in Fig. 3 dargestellte Herniennetz weist gegenüber der in Fig. 1 gezeigten Ausführungsform eine vergrößerte Brückenlasche 7 auf, die in ihrer Länge etwa der gesamten Längsseite 4 der Grundplatte 1" und in ihrer Breite etwa der halben Querseite 9 entspricht. Beim Einklappen der Brückenlasche 7 um die Faltkante 8 kommt sie im Bereich der Durchlassöffnung 3 zu liegen. Diesem Umstand wird durch eine halbkreisförmige Aussparung 10 in der Brückenlasche 7 Rechnung getragen. Im Übrigen ist wieder ein sich bis zum einen Ende der Brückenlasche 7 erstreckender Einführungsschlitz 5 in der Grundplatte 1" vorgesehen.

Eine zu Fig. 3 analoge Vergrößerung der Brückenlasche 7 zeigt das Herniennetz gemäß Fig. 4. Insoweit kann zu weiteren Ausführungen auf die Beschreibung der Fig. 3 verwiesen werden.

Ein weiterer Unterschied zum Ausführungsbeispiel gemäß Fig. 2 besteht in der Grundform der Grundplatte 1"', die hier wieder im Wesentlichen rechteckig ist.

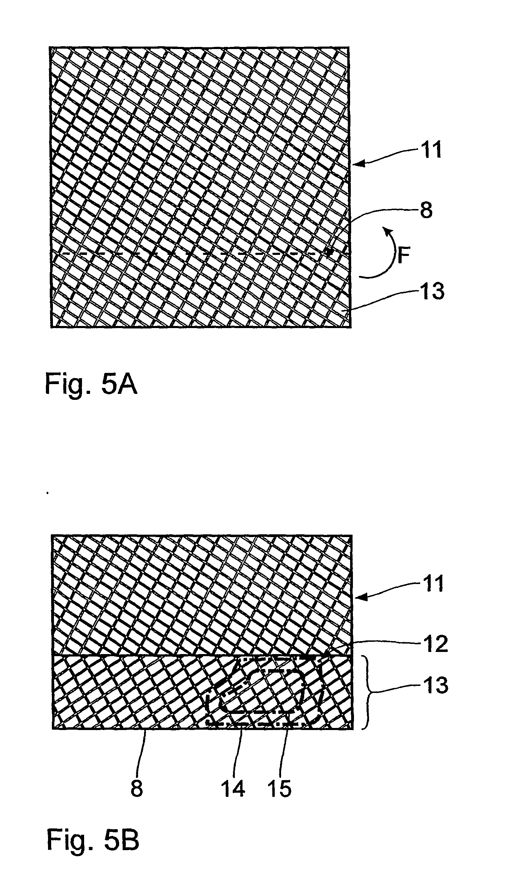

Anhand der Fig. 5A bis D wird nun die konstruktiv einfachen und geschickte Herstellung des in Fig. 3 gezeigten Hemiennetzes erläutert.

Es wird ausgegangen von einem rechteckigen Zuschnitt 11 des eingangs erwähnten Netzmaterials aus Polypropylen. Dieser Zuschnitt wird in einem ersten Schritt entlang einer strichliert gezeigten Falt-Linie 8 gefaltet (Pfeil F), die die Breite des Zuschnittes 11 im Verhältnis von etwas mehr als 2:1 teilt (Fig. 5A).

Anschließend wird mit Hilfe eines Polypropylen-Fadens, der dem Fadenmaterial des Zuschnittes 11 entspricht, eine geschlossene Doppelnaht 12 zwischen dem umgefalteten Laschenteil 13 und dem darunter befindlichen Netzmaterial des Zuschnittes 11 auf einer Halbseite des Umschlages angelegt. Die Positionierung der Doppelnaht 12 mit einer äußeren Naht 14 und einer mit Abstand davon nach innen versetzten inneren Naht 15 ist so gewählt, dass noch Platz für die Durchlassöffnung 3 und eine Randbesäumung des Zuschnittes 11 verbleibt. Das Herstellen der Doppelnaht 12 ist in Fig. 5B strichpunktiert angedeutet.

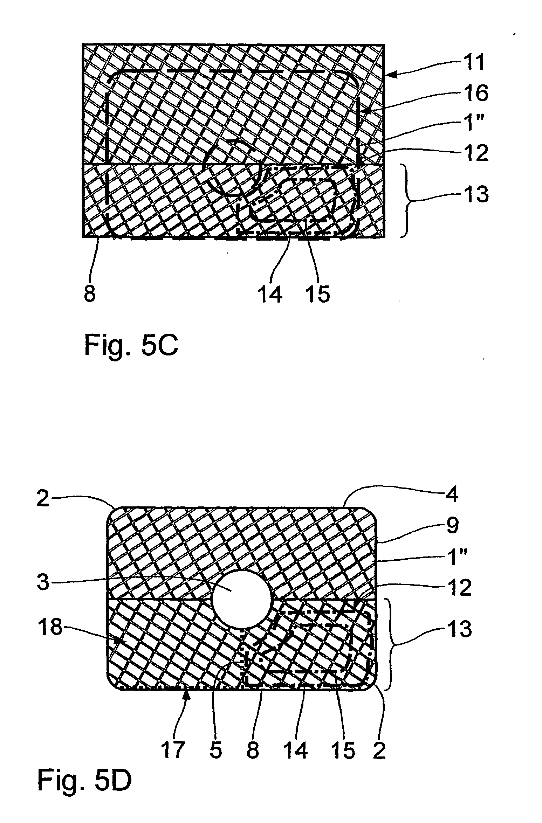

In einem nächsten Fertigungsschritt gemäß Fig. 5C wird- wie bereits angesprochen - durch Lasern oder Stanzen die eigentliche Kontur der Grundplatte 1" aus dem Zuschnitt 11 mit umgeklappten Laschenteil 13 herausgearbeitet, wie dies lang-strichliert angedeutet ist. Dabei ist darauf zu achten, dass die umlaufende, gegenüber dem Zuschnitt 1 etwas verkleinerte und in den Ecken abgerundete Laser- beziehungsweise Stanz-Schnittlinie 16 die Faltkante 8 zwischen Laschenteil 13 und Grundplatte 1" unversehrt lässt (Fig. 5C).

Das so herausgearbeitete Herniennetz wird noch durch einen Trennschnitt 17 zwischen Durchlassöffnung 3 und Faltkante 8 sowie entlang der Faltkante 8 zu der der Doppelnaht 12 abgewandten Seite hin komplettiert. Dieser in Fig. 5D punktiert angedeutete Trennschnitt 17 geht nur durch die die Grundplatte 1" bildende Lage des Netzmaterials, nicht jedoch durch das Laschenteil 13, sodass der links vom Trennschnitt 17 für den Einführungsschlitz 5 liegende Lappen des Laschenteils 13 nach wie vor am Zuschnitt 11 hängenbleibt und nach dem Einführen von Samenstrang oder Speiseröhre über den durch den Trennschnitt 17 gebildeten Einführungsschlitz 5 in die Durchlassöffnung 3 ein Vernähen dieses Lappens 18 mit dem darunter befindlichen Teil der Grundplatte 1" durch den Chirurgen vorgenommen werden kann.

Die Herniennetze gemäß den Fig. 1 bis 4 werden im Übrigen nach dem Zuschneiden aus einem Bahnmaterial mittels eines aus dem Stand der Technik bekannten PACVD-Prozesses mit einer die gesamte Netzoberfläche bedeckenden, durchgehenden Titanisierung versehen. Dieser Metallisierungsprozess ist beispielsweise aus der DE 199 45 299 A bekannt und führt zu einer titanhaltigen Beschichtung einer Dicke im Bereich von < 2 µm, vorzugsweise von 5 bis 700 nm. Praktische Werte der Beschichtungsdicke liegen bei 20 bis 30 nm.

## Patentansprüche

1. Herniennetz zur Versorgung von insbesondere Leisten- oder Hiatushernien, umfassend
- eine Grundplatte (1, 1', 1", 1"') aus einem lagenförmigen, flexiblen Netzmaterial,
- eine Durchlassöffnung (3) in der Grundplatte (1, 1', 1'', 1''') für eine Körperröhre, insbesondere für den Samenstrang oder die Speiseröhre,
- einen Einführungsschlitz (5) zwischen dem Umriss (4, 9) der Grundplatte (1, 1', 1", 1"') und der Durchlassöffnung (3) zum Einführen der Körperröhre in die Durchlassöffnung (3), und
- eine im Bereich der Mündung (6) des Einführungsschlitzes (5) liegende Nähbrücke (7), die über den Einführungsschlitz (5) klappbar und beiderseits davon mit dem Netzmaterial der Grundplatte (1, 1', 1",1'") vernähbar ist, **dadurch gekennzeichnet, dass** die Nähbrücke als einstückig mit dem Netzmaterial der Grundplatte (1, 1', 1", 1"') zugeschnittene Brückenlasche (7) ausgebildet ist.

2. Herniennetz nach Anspruch 1, **dadurch gekennzeichnet, dass** die Brückenlasche (7) eine rechteckige Grundform mit einer solchen Dimensionierung aufweist, dass in ihrem umgeklappten Zustand der Einführungsschlitz (5) bis mindestens kurz vor der Durchlassöffnung (3) überdeckt ist.

3. Herniennetz nach Anspruch 1 oder 2, **dadurch gekennzeichnet, dass** die Brückenlasche (7) den Einführungsschlitz (5) beiderseits im Wesentlichen symmetrisch überdeckt.

4. Herniennetz nach einem der Ansprüche 1 bis 3, **dadurch gekennzeichnet, dass** die Brückenlasche (7) unmittelbar anschließend an die Mündung (6) des Einführungsschlitzes (5) in die Umrisskante (4, 9) der Grundplatte (1, 1', 1'', 1''') einstückig an die Grundplatte (1,1', 1'', 1''') angesetzt ist.

5. Herniennetz nach einem der vorgenannten Ansprüche, **dadurch gekennzeichnet, dass** die Grundplatte (1, 1', 1'', 1''') und/oder die Brückenlasche (7) abgerundete Eckbereiche (2, 2') aufweisen.

6. Herniennetz nach einem der vorgenannten Ansprüche, **dadurch gekennzeichnet, dass** es aus einem Netz-Bahnmaterial vorzugsweise aus Polypropylen mit Hilfe eines Laser-Schneidstrahls zugeschnitten ist.

7. Herniennetz nach einem der vorgenannten Ansprüche, **gekennzeichnet durch** eine metallhaltige, durchgehende, körperverträgliche Beschichtung.

8. Herniennetz nach Anspruch 7, **dadurch gekennzeichnet, dass** die Beschichtung eine Titan-haltige Beschichtung mit einer Dicke von kleiner 2 µm, vorzugsweise von 5 bis 700 nm ist.

9. Herniennetz nach einem der vorgenannten Ansprüche, **dadurch gekennzeichnet, dass** die Brückenlasche (7) in einem Vorkonfektionierungszustand umgeklappt und auf einer Seite des Einführungsschlitzes (5) mit dem Netzmaterial der Grundplatte (1, 1', 1", 1"') vernäht ist.

10. Herniennetz nach Anspruch 9, **dadurch gekennzeichnet, dass** die einseitige Vernähung als Doppelnaht (12) mit einer äußeren Naht (14) und einer mit Abstand davon nach innen versetzten Naht (15) angelegt ist.

## Claims

1. A hernia mesh fabric for repair of in particular inguinal or hiatus hernias, comprising
- a base sheet (1, 1', 1", 1"') of layered, flexible mesh material;
- a passage (3) in the base sheet (1, 1', 1", 1"') for a body canal, in particular for the spermatic cord or the oesophagus;
- an insertion slit (5) between the contour (4, 9) of the base sheet (1, 1', 1", 1"') and the passage (3) for insertion of the body canal into the passage (3); and
- a sewing bridge (7) which is located in the vicinity of the mouth (6) of the insertion slit (5) and which is able to be folded down on the insertion slit (5) and, on both sides thereof, to be stitched to the mesh material of the base sheet (1,1',1",1"');
**characterized**
- **in that** the sewing bridge is a bridge tongue (7) which is cut to size in one piece with the mesh material of the base sheet (1, 1', 1", 1"').

2. A hernia mesh fabric according to claim 1, **characterized in that** the bridge tongue (7) has a rectangular basic shape of such dimensioning that, when it is doubled up, the insertion slit (5) is covered at least as far as slightly upstream of the passage (3).

3. A hernia mesh fabric according to claim 1 or 2, **characterized in that** the bridge tongue (7) covers the insertion slit (5) on both sides substantially symmetrically.

4. A hernia mesh fabric according to one of claims 1 to 3, **characterized in that** the bridge tongue (7) , where directly adjoining the mouth (6) of the insertion slit (5) into the contour (4, 9) of the base sheet (1, 1', 1", 1'"), is integrally attached to the base sheet (1, 1', 1", 1"').

5. A hernia mesh fabric according to one of the preceding claims, **characterized in that** the base sheet (1,1', 1", 1"') and/or the bridge tongue (7) have rounded corners (2, 2').

6. A hernia mesh fabric according to one of the preceding claims, **characterized in that** it is cut to size from meshed sheet material preferably of polypropylene by the aid of a laser cutting beam.

7. A hernia mesh fabric according to one of the preceding claims, **characterized by** a metal-containing, continuous, biocompatible coating.

8. A hernia mesh fabric according to claim 7, **characterized in that** the coating is a titanium-containing coating of a thickness of less than 2 µm, preferably of 5 to 700 nm.

9. A hernia mesh fabric according to one of the preceding claims, **characterized in that** the bridge tongue (7), in a condition of pre-fabrication, is doubled up and stitched to the mesh material of the base sheet (1,1', 1'', 1''') on one side of the insertion slit (5).

10. A hernia mesh fabric according to claim 9, **characterized in that** the unilateral stitching arrangement is a double-stitched seam (12), comprising an outer seam (14) and a seam (15) which is displaced inwards at a distance therefrom.

## Revendications

1. Filet herniaire servant à soigner en particulier des hernies hiatales ou inguinales hernies, comprenant
- une plaque de base (1, 1', 1'', 1''') conçue à partir d'un matériau de filet flexible en forme de couches,
- une ouverture de passage (3) dans la plaque de base (1, 1', 1", 1"') pour un canal corporel, en particulier pour le cordon spermatique ou l'oesophage,
- une fente d'introduction (5) entre le contour (4, 9) de la plaque de base (1, 1', 1'', 1"') et l'ouverture de passage (3) pour introduire le canal corporel dans l'ouverture de passage (3), et,
- un pont de couture (7) se trouvant dans le secteur de l'orifice (6) de la fente d'introduction (5) qui peut être rabattu sur la fente d'introduction (5) et être cousu des deux côtés de celle-ci avec le matériau du filet de la plaque de base (1, 1', 1", 1"') , **caractérisé en ce que** le pont de couture est formé comme un rabat de pont (7) découpé d'un seul tenant avec le matériau du filet la plaque de base (1, 1', 1" , 1"' ).

2. Filet herniaire selon la revendication 1, **caractérisé en ce que** le rabat du pont (7) comprend une forme de base rectangulaire avec un dimensionnement tel que à l'état rabattu, la fente d'introduction (5) est recouverte jusqu'à un point situé au moins peu avant l'ouverture de passage (3).

3. Filet herniaire selon la revendication 1 ou 2, **caractérisé en ce que** le rabat du pont (7) recouvre la fente d'introduction (5) des deux côtés de manière essentiellement symétrique.

4. Filet herniaire selon l'une quelconque des revendications 1 à 3, **caractérisé en ce que** le rabat du pont (7) est constitué directement à la suite de l'orifice (6) de la fente d'introduction (5) dans le bord du contour (4, 9) de la plaque de base (1, 1', 1" , 1"') d'un seul tenant sur la plaque de base (1, 1', 1", 1"').

5. Filet herniaire selon l'une quelconque des revendications susmentionnées, **caractérisé en ce que** la plaque de base (1, 1', 1'', 1''' ) et/ou le rabat du pont (7) possèdent des angles arrondis (2, 2').

6. Filet herniaire selon l'une quelconque des revendications susmentionnées, **caractérisé en ce qu'**il est découpé à partir d'un matériau de filet en bande, de préférence du polypropylène, à l'aide d'un rayon laser de découpe.

7. Filet herniaire selon l'une quelconque des revendications susmentionnées, **caractérisé par** un revêtement supporté par le corps, continu et à base de métaux.

8. Filet herniaire selon la revendication 7, **caractérisé en ce que** le revêtement à base de titane est d'une épaisseur inférieure à 2 µm, comprise de préférence entre 5 et 700 nm.

9. Filet herniaire selon l'une quelconque des revendications susmentionnées, **caractérisé en ce que** le rabat du pont (7) est rabattu dans un état de pré-confection et cousu sur un côté de la fente d'introduction (5) avec le matériau du filet de la plaque de base (1, 1', 1", 1''').

10. Filet herniaire selon la revendication 9, **caractérisé en ce que** la couture unilatérale est appliquée comme une couture double (12) avec une couture externe (14) et une couture interne décalé vers l'intérieur à une distance de celle-ci (15).
